# EUROPEAN PATENT APPLICATION

(11) **EP 3 848 050 A1**
(43) Date of publication of application: **14.07.2021**
(21) Application number: 19851221.2
(22) Date of filing: 21.08.2019
(51) Int. Cl.: A61K 45/00, A61K 31/513, A61K 31/7088, A61K 31/7105, A61K 31/713, A61K 33/243, A61P 35/00, A61P 43/00

(54) **MEDICINE USING HSP47 INHIBITOR TO ENHANCE SENSITIVITY TO CHEMOTHERAPEUTIC AGENT**

(30) Priority: 22.08.2018 JP 2018155147
(71) Applicant: Nitto Denko Corporation, Ibaraki-shi, Osaka 567-8680 (JP)
(72) Inventor: YONEDA, Akihiro, Sapporo-shi, Hokkaido 160-0808 (JP); TAMURA, Yasuaki, Sapporo-shi, Hokkaido 060-0808 (JP); TAKEI, Norio, Sapporo-shi, Hokkaido 060-0808 (JP); SAWADA, Kaori, Sapporo-shi, Hokkaido 060-0808 (JP)
(74) Representative: Hoffmann Eitle
(86) International application number: PCT/JP2019/032609
(87) International publication number: WO 2020/040185

(57) **Abstract**

Provided is a medicine containing a HSP47 inhibitor to increase sensitivity of a cancer patient to a chemotherapeutic agent.

SEQUENCE LISTING

<110>  NITTO DENKO CORPORATION

<120>
\202g\202r\202o\202S\202V\202Ì\221j\212Q\225¨\216¿\202ð\227p\202¢\202½\201A
\211»\212w\227Ã\226@\215Ü\212´\216ó\220«\202Ì\221\235\213-

<130>  PCT2954ND

<160>  9

<170>  PatentIn version 3.5

<210>  1
<211>  2208
<212>  RNA
<213>  Homo sapiens

<400>  1
ucuuuggcuu uuuuuggcgg agcuggggcg cccuccggaa gcguuuccaa cuuuccagaa      60

guuucucggg acgggcagga gggggugggg acugccauau auagaucccg ggagcagggg     120

agcgggcuaa gaguagaauc gugucgcggc ucgagagcga gagucacguc ccggcgcuag     180

cccagcccga cccaggccca ccguggugca cgcaaaccac uuccuggcca ugcgcucccu     240

ccugcuucuc agcgccuucu gccuccugga ggcggcccug gccgccgagg ugaagaaacc     300

ugcagccgca gcagcuccug gcacugcgga gaaguugagc cccaaggcgg ccacgcuugc     360

cgagcgcagc gccggccugg ccuucagcuu guaccaggcc auggccaagg accaggcagu     420

ggagaacauc cuggugucac ccgugguggu ggccucgucg cuagggcucg ugucgcuggg     480

cggcaaggcg accacggcgu cgcaggccaa ggcagugcug agcgccgagc agcugcgcga     540

cgaggaggug cacgccggcc ugggcgagcu gcugcgcuca cucagcaacu ccacggcgcg     600

caacgugacc uggaagcugg gcagccgacu guacggaccc agcucaguga gcuucgcuga     660

ugacuucgug cgcagcagca agcagcacua caacugcgag cacuccaaga ucaacuuccg     720

cgacaagcgc agcgcgcugc aguccaucaa cgagugggcc gcgcagacca ccgacggcaa     780

gcugcccgag gucaccaagg acguggagcg cacggacggc gcccugcuag ucaacgccau     840

guucuucaag ccacacuggg augagaaauu ccaccacaag augguggaca accguggcuu     900

cauggugacu cgguccuaua ccgugggugu caugaugaug caccggacag gccucuacaa     960

cuacuacgac gacgagaagg aaaagcugca aaucguggag augccccugg cccacaagcu    1020

cuccagccuc aucauccuca ugccccauca cguggagccu cucgagcgcc uugaaaagcu    1080

gcuaaccaaa gagcagcuga agaucuggau ggggaagaug cagaagaagg cuguugccau    1140

cuccuugccc aagggugugg uggaggugac ccaugaccug cagaaacacc uggcugggcu    1200

gggccugacu gaggccauug acaagaacaa ggccgacuug ucacgcaugu caggcaagaa    1260

ggaccuguac cuggccagcg uguuccacgc caccgccuuu gaguuggaca cagauggcaa    1320

ccccuuugac caggacaucu acgggcgcga ggagcugcgc agccccaagc uguucuacgc 1380

cgaccacccc uucaucuucc uagugcggga cacccaaagc ggcucccugc uauucauugg 1440

gcgccugguc cggccuaagg gugacaagau gcgagacgag uuauagggcc ucagggugca 1500

cacaggaugg caggaggcau ccaaaggcuc cugagacaca ugggugcuau ugggguuggg 1560

ggggagguga gguaccagcc uuggauacuc cauggggugg ggguggaaaa acagaccggg 1620

guucccgugu gccugagcgg accuucccag cuagaauuca cuccacuugg acaugggccc 1680

cagauaccau gaugcugagc ccggaaacuc cacauccugu gggaccuggg ccauagucau 1740

ucugccugcc cugaaagucc cagaucaagc cugccucaau caguauucau auuuauagcc 1800

agguaccuuc ucaccuguga gaccaaauug agcuaggggg gucagccagc ccucuucuga 1860

cacuaaaaca ccucagcugc cuccccagcu cuaucccaac cucucccaac uauaaaacua 1920

ggugcugcag ccccugggac caggcacccc cagaaugacc uggccgcagu gaggcggauu 1980

gagaaggagc ucccaggagg ggcuucuggg cagacucugg ucaagaagca ucgugucugg 2040

cguugugggg augaacuuuu uguuuuguuu cuuccuuuuu uaguucuuca aagauaggga 2100

gggaaggggg aacaugagcc uuuguugcua ucaauccaag aacuuauuug uacauuuuuu 2160

uuuucaauaa aacuuuucca augacauuuu guuggagcgu ggaaaaaa 2208

<210> 2
<211> 21
<212> DNA
<213> Homo sapiens

<400> 2
cuacgacgac gagaaggaat t 21

<210> 3
<211> 21
<212> DNA
<213> Homo sapiens

<400> 3
uuccuucucg ucgucguagt a 21

<210> 4
<211> 21
<212> DNA
<213> Homo sapiens

<400> 4
agcccucuuc ugacacuaat t 21

<210> 5
<211> 21
<212> DNA
<213> Homo sapiens

<400>  5
uuagugucag aagagggcug g                                                21

<210>  6
<211>  21
<212>  DNA
<213>  Homo sapiens

<400>  6
ggacaggccu cuacaacuat t                                                21

<210>  7
<211>  21
<212>  DNA
<213>  Homo sapiens

<400>  7
uaguuguaga ggccugucct t                                                21

<210>  8
<211>  23
<212>  DNA
<213>  Artificial Sequence

<220>
<223>  HSP47 gRNA

<400>  8
ccgactgtac ggacccagct cag                                              23

<210>  9
<211>  4140
<212>  DNA
<213>  Artificial Sequence

<220>
<223>  Cas9

<400>  9
atggacaaga agtactccat tgggctcgat atcggcacaa acagcgtcgg ctgggccgtc      60

attacggacg agtacaaggt gccgagcaaa aaattcaaag ttctgggcaa taccgatcgc     120

cacagcataa agaagaacct cattggcgcc ctcctgttcg actccgggga gacggccgaa     180

gccacgcggc tcaaaagaac agcacggcgc agatataccc gcagaaagaa tcggatctgc     240

tacctgcagg agatctttag taatgagatg gctaaggtgg atgactcttt cttccatagg     300

ctggaggagt cctttttggt ggaggaggat aaaaagcacg agcgccaccc aatctttggc     360

aatatcgtgg acgaggtggc gtaccatgaa aagtacccaa ccatatatca tctgaggaag     420

aagcttgtag acagtactga taaggctgac ttgcggttga tctatctcgc gctggcgcat     480

atgatcaaat ttcggggaca cttcctcatc gagggggacc tgaacccaga caacagcgat     540

gtcgacaaac tctttatcca actggttcag acttacaatc agcttttcga agagaacccg     600

atcaacgcat ccggagttga cgccaaagca atcctgagcg ctaggctgtc caaatcccgg 660

cggctcgaaa acctcatcgc acagctccct ggggagaaga agaacggcct gtttggtaat 720

cttatcgccc tgtcactcgg gctgaccccc aactttaaat ctaacttcga cctggccgaa 780

gatgccaagc ttcaactgag caaagacacc tacgatgatg atctcgacaa tctgctggcc 840

cagatcggcg accagtacgc agaccttttt ttggcggcaa agaacctgtc agacgccatt 900

ctgctgagtg atattctgcg agtgaacacg gagatcacca aagctccgct gagcgctagt 960

atgatcaagc gctatgatga gcaccaccaa gacttgactt tgctgaaggc ccttgtcaga 1020

cagcaactgc ctgagaagta caaggaaatt ttcttcgatc agtctaaaaa tggctacgcc 1080

ggatacattg acggcggagc aagccaggag gaattttaca aatttattaa gcccatcttg 1140

gaaaaaatgg acggcaccga ggagctgctg gtaaagctta acagagaaga tctgttgcgc 1200

aaacagcgca ctttcgacaa tggaagcatc ccccaccaga ttcacctggg cgaactgcac 1260

gctatcctca ggcggcaaga ggatttctac ccctttttga aagataacag ggaaaagatt 1320

gagaaaatcc tcacatttcg gataccctac tatgtaggcc ccctcgcccg gggaaattcc 1380

agattcgcgt ggatgactcg caaatcagaa gagaccatca ctccctggaa cttcgaggaa 1440

gtcgtggata agggggcctc tgcccagtcc ttcatcgaaa ggatgactaa ctttgataaa 1500

aatctgccta acgaaaaggt gcttcctaaa cactctctgc tgtacgagta cttcacagtt 1560

tataacgagc tcaccaaggt caaatacgtc acagaaggga tgagaaagcc agcattcctg 1620

tctggagagc agaagaaagc tatcgtggac ctcctcttca agacgaaccg gaaagttacc 1680

gtgaaacagc tcaaagaaga ctatttcaaa aagattgaat gtttcgactc tgttgaaatc 1740

agcggagtgg aggatcgctt caacgcatcc ctgggaacgt atcacgatct cctgaaaatc 1800

attaaagaca aggacttcct ggacaatgag gagaacgagg acattcttga ggacattgtc 1860

ctcaccctta cgttgtttga agatagggag atgattgaag aacgcttgaa aacttacgct 1920

catctcttcg acgacaaagt catgaaacag ctcaagaggc gccgatatac aggatggggg 1980

cggctgtcaa gaaaactgat caatgggatc cgagacaagc agagtggaaa gacaatcctg 2040

gattttctta agtccgatgg atttgccaac cggaacttca tgcagttgat ccatgatgac 2100

tctctcacct ttaaggagga catccagaaa gcacaagttt ctggccaggg ggacagtctt 2160

cacgagcaca tcgctaatct tgcaggtagc ccagctatca aaaagggaat actgcagacc 2220

gttaaggtcg tggatgaact cgtcaaagta atgggaaggc ataagcccga gaatatcgtt 2280

atcgagatgg cccgagagaa ccaaactacc cagaagggac agaagaacag tagggaaagg 2340

atgaagagga ttgaagaggg tataaaagaa ctggggtccc aaatccttaa ggaacaccca 2400

gttgaaaaca cccagcttca gaatgagaag ctctacctgt actacctgca gaacggcagg 2460

gacatgtacg tggatcagga actggacatc aatcggctct ccgactacga cgtggatcat 2520

atcgtgcccc agtcttttct caaagatgat tctattgata ataaagtgtt gacaagatcc 2580

gataaaaata gagggaagag tgataacgtc ccctcagaag aagttgtcaa gaaaatgaaa 2640

aattattggc ggcagctgct gaacgccaaa ctgatcacac aacggaagtt cgataatctg 2700

actaaggctg aacgaggtgg cctgtctgag ttggataaag ccggcttcat caaaaggcag 2760

cttgttgaga cacgccagat caccaagcac gtggcccaaa ttctcgattc acgcatgaac 2820

accaagtacg atgaaaatga caaactgatt cgagaggtga aagttattac tctgaagtct 2880

aagctggtct cagatttcag aaaggacttt cagttttata aggtgagaga gatcaacaat 2940

taccaccatg cgcatgatgc ctacctgaat gcagtggtag gcactgcact tatcaaaaaa 3000

tatcccaagc ttgaatctga atttgtttac ggagactata aagtgtacga tgttaggaaa 3060

atgatcgcaa agtctgagca ggaaataggc aaggccaccg ctaagtactt cttttacagc 3120

aatattatga attttttcaa gaccgagatt acactggcca atggagagat tcggaagcga 3180

ccacttatcg aaacaaacgg agaaacagga gaaatcgtgt gggacaaggg tagggatttc 3240

gcgacagtcc ggaaggtcct gtccatgccg caggtgaaca tcgttaaaaa gaccgaagta 3300

cagaccggag gcttctccaa ggaaagtatc ctcccgaaaa ggaacagcga caagctgatc 3360

gcacgcaaaa aagattggga ccccaagaaa tacggcggat tcgattctcc tacagtcgct 3420

tacagtgtac tggttgtggc caaagtggag aaagggaagt ctaaaaaact caaaagcgtc 3480

aaggaactgc tgggcatcac aatcatggag cgatcaagct tcgaaaaaaa ccccatcgac 3540

tttctcgagg cgaaaggata taaagaggtc aaaaaagacc tcatcattaa gcttcccaag 3600

tactctctct ttgagcttga aaacggccgg aaacgaatgc tcgctagtgc gggcgagctg 3660

cagaaaggta acgagctggc actgccctct aaatacgtta atttcttgta tctggccagc 3720

cactatgaaa agctcaaagg gtctcccgaa gataatgagc agaagcagct gttcgtggaa 3780

caacacaaac actaccttga tgagatcatc gagcaaataa gcgaattctc caaaagagtg 3840

atcctcgccg acgctaacct cgataaggtg ctttctgctt acaataagca cagggataag 3900

cccatcaggg agcaggcaga aaacattatc cacttgttta ctctgaccaa cttgggcgcg 3960

cctgcagcct tcaagtactt cgacaccacc atagacagaa agcggtacac ctctacaaag 4020

gaggtcctgg acgccacact gattcatcag tcaattacgg ggctctatga aacaagaatc 4080

gacctctctc agctcggtgg agacagcagg gctgacccca agaagaagag gaaggtgtga 4140

## Description

### [Technical Field]

The present invention relates to a medicine and a method for enhancing the sensitivity of a cancer patient to a chemotherapeutic agent.

### [Background Art]

Regarding therapeutic methods for cancer, surgical therapy, radiation therapy, particle beam therapy, chemotherapy, molecular targeted therapy, and the like are available. Among these, treatment by means of chemotherapeutic agents has taken an important position in cancer treatment; however, there are cancers having resistance to chemotherapeutic agents, and side effects may occur when large amounts of a chemotherapeutic agent are administered.

With regard to cancer treatment, combination therapies in which one or more kinds of chemotherapeutic agents having different advantages in combination are also implemented. In combination therapies for cancer, for example, cisplatin and 5-fluorouracil or the like are used. However, there are still numerous cancers that are difficult to treat with chemotherapeutic agents, and there is a demand for new treatment methods.

In Patent Document 1, a method for treating a malignant tumor using a molecule that targets HSP47 is described; however, it is not described whether a molecule targeting HSP47 can increase the sensitivity of a cancer patient to a chemotherapeutic agent.

### [Citation List]

Patent Document 1: U.S. Patent Publication No. 2017/0218365

### [Summary of the Invention]

### Problems to be solved by the Invention

An object of the invention is to enhance the sensitivity of a cancer patient to a chemotherapeutic agent.

### Means for Solving the Problems

The inventors of the invention repeatedly conducted a thorough investigation in order to address the above-described problems, and they found that in a case where a molecule targeting HSP47 and cisplatin or 5-fluorouracil are administered, proliferation of various cancer cells can be conspicuously suppressed, that is, the molecule targeting HSP47 can enhance the sensitivity of a cancer patient to a chemotherapeutic agent, as compared to the case of administering the molecule targeting HSP47 alone, or as compared to the case of administering cisplatin or 5-fluorouracil alone, thus completing the invention. Chemotherapeutic agents having actions such as DNA synthesis inhibition and DNA damage can induce cell death by inducing endoplasmic reticulum stress. An HSP47 inhibitor is expected to inhibit the effect exerted by HSP47 for alleviating endoplasmic reticulum stress and to increase the intracellular endoplasmic reticulum stress.

That is, the invention relates to the following.
(1) A medicine for increasing sensitivity of a cancer patient to a chemotherapeutic agent, comprising an HSP47 inhibitor.
(2) The medicine according to (1), wherein the HSP47 inhibitor is an interfering nucleic acid against HSP47, a ribozyme, an antisense nucleic acid, a microRNA, a short hairpin RNA, a vector expressing any of these, or a cell transformed with any of these.
(3) The medicine according to (2), wherein the interfering nucleic acid against HSP47 is a siNA or a siRNA.
(4) The medicine according to any one of (1) to (3), wherein the cancer patient is a patient with breast cancer, colorectal cancer, colon cancer, or pancreatic cancer.
(5) The medicine according to any one of (1) to (4), wherein the chemotherapeutic agent is an alkylating agent, an antimetabolite, an antitumor antibiotic substance, an alkaloid, a hormonal therapeutic agent, a platinum complex, an angiogenesis inhibitory agent, a topoisomerase inhibitory agent, or a microtubule agonist.
(6) The medicine according to any one of (1) to (5), wherein the chemotherapeutic agent is cisplatin (CDDP) or 5-fluorouracil (5-FU).
(7) A medicine for treating cancer, comprising an HSP47 inhibitor and a chemotherapeutic agent.
(8) Use of an HSP47 inhibitor in production of a medicine for the treatment of cancer, the treatment being a treatment of administering a chemotherapeutic agent and an HSP47 inhibitor in combination.
(9) A composition comprising an HSP47 inhibitor for use in the treatment of cancer, the treatment being a treatment of administering a chemotherapeutic agent and an HSP47 inhibitor in combination.
(10) A method for increasing sensitivity of a cancer patient to a chemotherapeutic agent, comprising administering an effective amount of an HSP47 inhibitor to a cancer patient.
(11) The method according to (10), further comprising a step of identifying a patient in need of treatment using a medicine comprising an HSP47 inhibitor.
(12) The method according to (11), wherein the step is a step of quantitatively determining the HSP47 expression in a tumor tissue isolated from a cancer patient.
(13) A method for treating cancer, comprising administering effective amounts of an HSP47 inhibitor and a chemotherapeutic agent to a cancer patient.
(14) A method for treating cancer cells in vitro, comprising treating cancer patient-derived cancer cells in vitro using effective amounts of an HSP47 inhibitor and a chemotherapeutic agent.

### Advantageous Effects of the Invention

The medicine according to the invention can enhance the sensitivity of various cancer patients to a chemotherapeutic agent by inhibiting the expression of HSP47 (including the expression of both or either of HSP47 mRNA and protein) in cancer cells.

Through the combination therapy of the invention, proliferation of various cancer cells can be efficiently suppressed, and cancer can be treated or cured.

According to the invention, the amount of administration of a chemotherapeutic agent can be reduced to a large extent in cancer treatment, and anticancer treatment can be carried out while reducing the risk of side effects caused by the chemotherapeutic agent.

### [Brief Description of the Drawings]

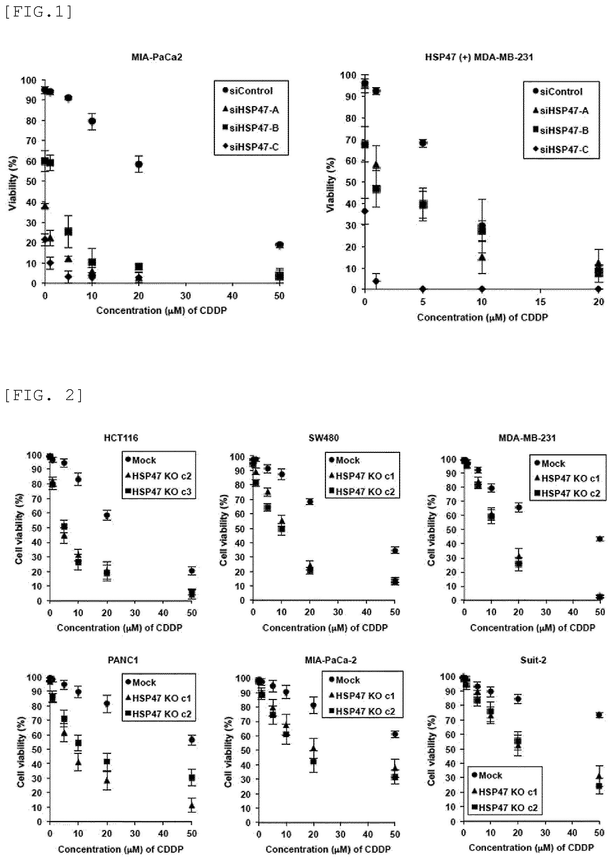

[FIG. 1] Fig. 1 shows the viability of human cancer cells with HSP47 silenced, after treating the human cancer cells with cisplatin (CDDP). MIA-PaCa-2 cells and MDA-MB-231 cells were transfected with control siRNA (siControl, 10 nM) and HSP47 siRNA (siHSP47, 10 nM) in any one of three batches (A, B, and C) and were cultured for 48 hours. After 48 hours from the transfection with siRNA, the cells were treated with cisplatin (CDDP) at a concentration of 0, 1, 5, 10, 20, or 50 µM and were cultured for 24 hours. After 24 hours from the treatment with CDDP, the viability of the cells was determined by a Dye-exclusion assay. The axis of ordinate represents the cell viability (%), and the axis of abscissa represents the concentration (µM) of cisplatin.
[FIG. 2] Fig. 2 shows the viability of HSP47 knockout human cancer cells treated with CDDP. Human cancer cells (Mock) and HSP47 knockout (KO) cancer cells were treated with cisplatin (CDDP) at a concentration of 0, 1, 5, 10, 20, or 50 µM. After 24 hours from the treatment with CDDP, the viability of the cells was determined by a dye-exclusion assay. The axis of ordinate represents the cell viability (%), and the axis of abscissa represents the concentration (µM) of cisplatin.

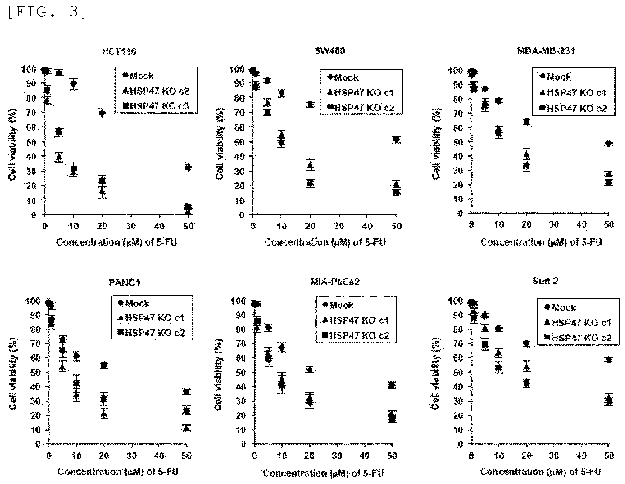

[FIG. 3] Fig. 3 shows the viability of HSP47 knockout human cancer cells treated with 5-FU. Human cancer cells (Mock) and HSP47 knockout (KO) cancer cells were treated with 5-fluorouracil (5-FU) at a concentration of 0, 1, 5, 10, 20, or 50 µM. After 24 hours from the treatment with 5-FU, the viability of the cells was determined by a dye-exclusion assay. The axis of ordinate represents the cell viability (%), and the axis of abscissa represents the concentration (µM) of 5-fluorouracil.

### [Description of Embodiments]

In the present specification, a medicine comprising an HSP47 inhibitor for increasing the sensitivity of a cancer patient to a chemotherapeutic agent; a method and a kit using this medicine; a method for enhancing the sensitivity to a chemotherapeutic agent, the method comprising administering an HSP47 inhibitor (hereinafter, may be referred to as chemotherapeutic agent sensitivity enhancing method); and a method for treating cancer, the method comprising administering an HSP47 inhibitor and a chemotherapeutic agent (hereinafter, may be referred to as combination therapy) are provided.

The medicine, method, and kit according to the invention are characterized in using a nucleic acid molecule (for example, small interfering nucleic acid (siNA), small interfering RNA (siRNA), double-stranded RNA (dsRNA), microRNA (miRNA), or short hairpin RNA (shRNA)) that binds to transcript RNA (including pre-mRNA and mRNA) of HSP47, for example, transcript RNA of HSP47 as set forth in SEQ ID NO:1, or knocking out the HSP47 gene using a genome editing technology, for example, CRISPR-Cas9, which utilizes single-stranded guide RNA (sgRNA, gRNA) (for example, SEQ ID NO: 8) specific to the HSP47 genome.

Furthermore, the medicine, method, and kit according to the invention are characterized in using a chemotherapeutic agent in combination, in addition to using the above-described nucleic acid molecule (for example, small interfering nucleic acid (siNA), small interfering RNA (siRNA), double-stranded RNA (dsRNA), microRNA (miRNA), or short hairpin RNA (shRNA)) that binds to transcript RNA of HSP47, or knocking out the HSP47 gene using a genome editing technology, for example, CRISPR-Cas9, which utilizes single-stranded guide RNA (sgRNA, gRNA) (for example, SEQ ID NO: 8) specific to the HSP47 genome.

### 1. Medicine of invention

An aspect of the invention relates to a medicine for enhancing the sensitivity of a cancer patient to a chemotherapeutic agent, the medicine comprising an HSP47 inhibitor, for example, an inhibitory agent for the expression of HSP47.

The base sequence and amino acid sequence of the HSP47 gene are known in the pertinent art, and in this invention, the mRNA sequence of HSP47 is set forth in SEQ ID NO:1.

According to the invention, the transcript RNA of HSP47 may be, for example, derived from a human, and may have a base sequence set forth in SEQ ID NO:1 or the same base sequence except that one to several nucleotides are deleted, substituted, added, or inserted, or a base sequence having a sequence identity of 70% or higher, 80% or higher, or 90% or higher, preferably 95% or higher, and more preferably 98% or higher or 99% or higher, with each of the above-described base sequences.

In the present specification, the term "several" means a number of bases of 2 to 10, preferably 2 to 5, and more preferably 2 or 3. Furthermore, the sequence identity can be determined using, for example, a known algorithm such as BLAST.

According to the invention, the inhibitory agent for the expression of HSP47 may be a component that inhibits the expression of HSP47 or a component that inhibits the functions of HSP47 protein, and examples include a siRNA having RNA interference (RNAi) action (for example, containing siHSP47-A: 5'-CUACGACGACGAGAAGGAAtt-3' (SEQ ID NO:2), siHSP47-B: 5'-AGCCCUCUUCUGACACUAAtt-3' (SEQ ID NO:4), or siHSP47-C: 5'-GGACAGGCCUCUACAACUAtt-3' (SEQ ID NO:6) of the sense strand, or containing siHSP47-A: 5'-UUCCUUCUCGUCGUCGUAGta-3' (SEQ ID NO:3), siHSP47-B: 5'-UUAGUGUCAGAAGAGGGCUgg-3' (SEQ ID NO:5), or siHSP47-C: 5'-UAGUUGUAGAGGCCUGUCCtt-3' (SEQ ID NO:7) of the antisense strand)) or a precursor RNA thereof (for example, shRNA), or a modified RNA thereof, or a vector containing a DNA encoding a siRNA against the transcript RNA of the HSP47 gene. Furthermore, according to the invention, the inhibitory agent for the expression of HSP47 may be, for example, a siNA, a ribozyme, a shRNA, or a miRNA. The vector may contain an antisense RNA, an antisense DNA, a DNA encoding the antisense RNA, or an antisense DNA thereof. According to the invention, it is preferable that the vector contains, for example, a sequence of 5'-CCGACTGTACGGACCCAGCTCAG-3' (SEQ ID NO:8), and it is preferable that a vector containing the sequence of SEQ ID NO:8 further contains a Cas9 sequence (SEQ ID NO:9).

According to the invention, the siRNA may be a double-stranded RNA composed of sense RNA and antisense RNA, the double-stranded RNA having an antisense RNA containing 18 to 25 nucleotides, preferably 20 to 24 nucleotides, and more preferably 21 to 23 nucleotides, which is substantially complementary to a portion of the transcript RNA of the HSP47 gene, and having RNAi (RNA interference) action.

According to the invention, the term "complementary" means that a nucleic acid can form hydrogen bonds with another nucleic acid sequence by the classical Watson-Crick type or other non-classical type.

Furthermore, according to the invention, the term "substantially complementary" includes not only a case where all the contiguous residues of a nucleic acid sequence form hydrogen bonds with the same number of contiguous residues in another nucleic acid sequence, but also a case where, for example, 70%, 80%, and 90% of residues among all the residues of a nucleic acid sequence form hydrogen bonds with the residues of another nucleic acid sequence.

Therefore, according to the invention, the siRNA may have an antisense RNA containing a nucleotide obtained by modifying several bases of a nucleotide that is 100% complementary to a portion of the transcript RNA of the HSP47 gene.

Furthermore, according to the invention, the 3'-termini of the sense RNA and the antisense RNA may each have a protruding end of 2 to 5 nucleotides, and preferably two nucleotides. According to the invention, the siRNA may be a modified siRNA.

According to the invention, the HSP47 inhibitor may be, for example, a combination of siRNA containing a sense strand and an antisense strand, and for example, the following combinations are preferred.

Combination (siHSP47-A) of 5'-CUACGACGACGAGAAGGAAtt-3' (SEQ ID NO:2) of the sense strand and 5'-UUCCUUCUCGUCGUCGUAGta-3' (SEQ ID NO:3) of the antisense strand (Ambion)

Combination (siHSP47-B) of 5'-AGCCCUCUUCUGACACUAAtt-3' (SEQ ID NO:4) of the sense strand and 5'-UUAGUGUCAGAAGAGGGCUgg-3' (SEQ ID NO:5) of the antisense strand (Ambion)

Combination (siHSP47-C) of 5'-GGACAGGCCUCUACAACUAtt-3' (SEQ ID NO:6) of the sense strand and 5'-UAGUUGUAGAGGCCUGUCCtt-3' (SEQ ID NO:7) of the antisense strand (created by Nitto Denko Corporation)

Regarding the introduction of an HSP47 inhibitor into cells, examples of a known technique for introduction that can be used include, without limitations, a lipofectamine method, a lipofection method, a calcium phosphate method, an ultrasonic introduction method, an electroporation method, a particle gun method, a method of utilizing a viral vector (for example, an adenovirus vector, an adeno-associated virus vector, or a retrovirus vector), and a microinjection method.

In the case of using a viral vector, the viral titer may be 1x10³ to 1x10¹⁵ p.f.u. (plaque-forming unit), and the viral vector can be used at a viral titer of preferably 1×10⁵ to 1×10¹³, more preferably 1×10⁷ to 1×10¹¹, and even more preferably 1×10⁸ to 1×10¹⁰.

The nucleic acid molecule may be used as a naked nucleic acid or may be used after being incorporated into various nucleic acid constructs or vectors. Regarding the vectors, any known vectors such as a plasmid vector, a phage vector, a phagemid vector, a cosmid vector, and a viral vector can be utilized. It is preferable that the nucleic acid constructs or vectors include at least appropriate transcriptional or translational regulatory sequences derived from, for example, mammalian, microbial, viral, or insect genes. Such regulatory sequences include a sequence having a regulatory role for gene expression, for example, a transcription promoter or enhancer, an operator sequence for regulating transcription, a sequence encoding a ribosome binding site within messenger RNA, and a sequence appropriate for regulating transcription, translation initiation, or transcription termination.

Since the medicine according to the invention includes the above-described HSP47 inhibitor, the medicine can enhance the sensitivity of a cancer patient to a chemotherapeutic agent and is useful as an active ingredient for a medicine. The medicine according to the invention may include a chemotherapeutic agent in addition to an HSP47 inhibitor. The medicine according to the invention may include the above-described HSP47 inhibitor and one or two or more of pharmaceutically acceptable surfactants, carriers, diluents, and/or excipients. The pharmaceutically acceptable carriers, diluents, and the like are well known in the medical field and are described in, for example, Remington's Pharmaceutical Sciences, 18th Ed., Mack Publishing Co., Easton, PA (1990), the entire disclosure of which is incorporated herein by reference.

The medicine can be used for, for example, an enhancement in sensitivity to a chemotherapeutic agent, and more particularly, cancer treatment carried out concomitantly with an enhancement in sensitivity to a chemotherapeutic agent, depending on the constituent elements of the medicine.

Whether the sensitivity to a chemotherapeutic agent has increased when an HSP47 inhibitor is used in combination, is determined by considering whether a 50% viability can be induced by a lower concentration of the chemotherapeutic agent when this concentration is compared with the concentration of the chemotherapeutic agent required to induce a cancer cell viability of 50% without using the HSP47 inhibitor. When a relative value of the concentration of the chemotherapeutic agent required to induce a cancer cell viability of 50% without using the HSP47 inhibitor is designated as 100, in a case where the relative value of the chemotherapeutic agent concentration is less than 100, it can be said that sensitivity has increased, and in a case where the relative value is less than 50, it can be said that sensitivity has markedly increased. When the relative value of the chemotherapeutic agent concentration is less than 50, anticancer treatment can be carried out while reducing the risk of side effects caused by the chemotherapeutic agent.

The cancer cell viability represents a relative value (%) of the number of surviving cancer cells when the number of cancer cells before treatment with an HSP47 inhibitor or a chemotherapeutic agent is designated as 100%, and can be measured by a Dye-exclusion assay. The Dye-exclusion assay enables measurement of the number of surviving cells by, for example, determining the life or death of a cell on the basis of the presence or absence of trypan blue staining. The dye-exclusion method based on trypan blue staining can be carried out by, for example, mixing equal amounts of a 0.4% trypan blue solution (selling agency: FUJIFILM Wako Pure Chemical Corporation, product code: 20717081) and a cell suspension obtained by suspending cells in a culture medium, and counting the number of stained cells and unstained cells on a hemocytometer.

The cancer for which the invention is intended is not limited so long as the cancer expresses transcript RNA of HSP47, and examples include sarcomas such as fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, and osteosarcoma; carcinomas such as brain tumor, head and neck cancer, breast cancer, lung cancer, esophageal cancer, stomach cancer, duodenal cancer, appendiceal cancer, colorectal cancer, rectal cancer, colon cancer, liver cancer, pancreatic cancer, gallbladder cancer, bile duct cancer, anal cancer, kidney cancer, ureteral cancer, urinary bladder cancer, prostate cancer, penile cancer, testicular cancer, uterine cancer, ovarian cancer, vulvar cancer, vaginal cancer, and skin cancer; leukemia, and malignant lymphoma. Among these, solid tumors are preferable, and breast cancer, colorectal cancer, colon cancer, lung cancer, and pancreatic cancer are more preferable.

Whether cancer cells express HSP47 can be determined by detecting the expression of transcript RNA of HSP47 at the gene level. Specifically, at the gene level, for example, detection can be made by any known gene expression analysis method such as a Northern blotting method, an RNase protection assay, PCR methods such as RT-PCR and real-time PCR, an in situ hybridization method, or an in vitro transcription method, and detection by real-time PCR is preferable. Furthermore, whether cancer cells express HSP47 can be determined by detecting the expression of HSP47 at the protein level. Specifically, for example, an immunoprecipitation method, EIA (enzyme immunoassay) (for example, ELISA (enzyme-linked immunosorbent assay)), RIA (radioimmunoassay) (for example, IRMA (Immunoradiometric assay), RAST (radioallergosorbent test), and RIST (radioimmunosorbent test)), a Western blotting method, an immunohistochemistry method, an immunocytochemistry method, or a flow cytometry method can be used for detection. The transcript RNA of HSP47 is expressed in cancer cells; however, generally, the transcript RNA of HSP47 is substantially not expressed in normal cells, except for fibroblasts and myofibroblasts. According to the invention, cancer cells express HSP47 protein.

According to an embodiment of the invention, the medicine of the invention includes an HSP47 inhibitor for increasing the sensitivity of a cancer patient to a chemotherapeutic agent. According to another embodiment of the invention, the medicine of the invention may further include another chemotherapeutic agent that is useful for treating a target disease.

Regarding the chemotherapeutic agent, a chemotherapeutic agent used for cancer treatment, such as a cell-permeable anticancer agent, is preferable, and as the cell-permeable anticancer agent, without limitations, a chemotherapeutic agent having cell damaging action such as cytocidal action and proliferation inhibitory action, for example, an alkylating agent, an antimetabolite (for example, 5-fluorouracil or gemcitabine), an antitumor antibiotic substance, an alkaloid, a hormonal therapeutic agent, a platinum complex (for example, cisplatin), an angiogenesis inhibitory agent, a topoisomerase inhibitory agent, and a microtubule agonist may be included. Among these, an alkylating agent, an antimetabolite, an antitumor antibiotic substance, a platinum complex, or a topoisomerase is a chemotherapeutic agent capable of inducing DNA synthesis inhibition and DNA damage and inducing endoplasmic reticulum stress, and is preferable from the viewpoint that when the chemotherapeutic agent is used in combination of an HSP47 inhibitor, the anticancer action by the chemotherapeutic agent is particularly enhanced. For the medicine according to the invention, a combination of an HSP47 inhibitor and cisplatin or 5-fluorouracil is most preferred. In the case of administering these in combination, a preferable concentration of the HSP47 inhibitor is 1 nM to 100 nM as the final concentration, and a preferable concentration of cisplatin and 5-fluorouracil is 5 µM to 100 µM.

In a case where an HSP47 inhibitor and a chemotherapeutic agent are used in combination for the medicine according to the invention, these may be included in a single composition or may be included separately in a plurality of compositions, and those compositions may be separately used or may be used in combination.

According to various embodiments of the invention, the above-described medicine can be used in a state of being carried on various drug delivery carriers. Such carriers are not limited, and examples include polymer nanoparticles, polymicelles, dendrimers, liposomes, virus nanoparticles, and carbon nanotubes (see Cho K. et al., Clin Cancer Res. 2008 Mar 1;14 (5) :1310-6, and the like).

The medicine according to the invention may be administered by various routes including both oral and parenteral routes, examples of which include, without limitations, routes such as oral, intravenous, intramuscular, subcutaneous, topical, rectal, intratumoral, intraarterial, intraportal, intramedullary, intrapulpal, sublingual, intraoral, intraventricular, transmucosal, transdermal, intranasal, intraperitoneal, intrapulmonary, and intrauterine routes, and the medicine may be formulated into a dosage form appropriate for each of the administration routes . Regarding such a dosage form and preparation method, any known dosage form and method can be appropriately employed (see, for example, Standard Pharmaceutics, edited by Watanabe Yoshiteru, et al., Nankodo, 2003; and Remington's Pharmaceutical Sciences).

Examples of a dosage form appropriate for oral administration include, without limitations, a powder, a granular preparation, a tablet, a capsule, a liquid preparation, a suspension, an emulsion, a gel preparation, and a syrup preparation, and examples of a dosage form appropriate for parenteral administration include injectable preparations such as a solution injectable preparation, a suspension injectable preparation, an emulsion injectable preparation, and a prepared-upon-use type injectable preparation. The preparation for parenteral administration can be in the form of an aqueous or non-aqueous isotonic sterile solution or suspension.

### 2. Kit of invention

The invention also relates to a preparation kit of compositions comprising one or two or more containers that include an active ingredient (for example, HSP47 inhibitor) that can be included in the medicine according to the invention and a chemotherapeutic agent as necessary, singly or in combination; and necessary constituent elements of a medicine provided in the form of such a kit. The kit according to the invention may include, in addition to those described above, instructions in which a preparation method, administration method, and the like of the medicine according to the invention, for example, an instruction manual or an electronic recording medium such as a CD or a DVD.

### 3. Method of invention

An aspect of the invention relates to a method for increasing the sensitivity of a cancer patient to a chemotherapeutic agent, the method comprising administering an HSP47 inhibitor (chemotherapeutic agent sensitivity enhancing method).

Another aspect of the invention relates to a method for treating cancer, the method comprising administering an HSP47 inhibitor and a chemotherapeutic agent (combination therapy).

With regard to the methods according to the invention, the patient who receives administration is typically a cancer patient in need of treatment by means of a chemotherapeutic agent. Examples of such a patient include, without limitations, patients who are suffering from the above-described cancers related to high expression of HSP47, who have been diagnosed to have these cancers, or who have a high risk of developing these cancers. Therefore, the methods according to the invention may further include a step of identifying a patient in need of treatment using the medicine according to the invention, the medicine comprising an HSP47 inhibitor. The above-described step is, for example, a step of quantitatively determining the expression of mRNA or protein of HSP47 in a tumor tissue isolated from a cancer patient. Specific examples of the cancer are as described above in relation to the medicine.

With regard to the methods according to the invention, the medicine according to the invention can be used in combination with another active substance or treatment method useful for treating the target cancer. The methods according to the invention can be used in combination with physical therapy such as radiation treatment, surgical treatment such as surgical operation, or the like as the treatment method. In the case of treating a cancer patient by surgical treatment, the methods can be applied to either or both of the preoperative chemotherapy and the postoperative chemotherapy of the invention.

An effective amount in the therapeutic or treatment method according to the invention is, for example, an amount with which symptoms of a disease are reduced, or the progression of the disease is delayed or stopped, and preferably, the effective amount is an amount with which a disease is suppressed or cured. Furthermore, an amount that does not cause adverse effects exceeding the benefit of administration is preferable. Such an amount can be appropriately determined by an in vitro test using cultured cells or the like, or a test with a model animal such as a mouse, a rat, a dog, or a pig, and such testing methods are well known to those ordinarily skilled in the art. Furthermore, the dosage of the drug that is used for the treatment method according to the invention is known to those ordinarily skilled in the art or can be appropriately determined by the above-described tests or the like. According to the invention, a preferable concentration of a chemotherapeutic agent such as cisplatin and 5-fluorouracil is 5 µM to 100 µM.

Specific dosage of the active ingredient (for example, HSP47 inhibitor) that is administered in the methods according to the invention as described in the present specification can be determined by considering various conditions related to the subject who requires treatment, for example, severity of the symptoms, general health condition of the subject, age, body weight, gender of the subject, diet, timing and frequency of administration, medicine that is being used in combination, responsiveness to therapy, dosage form, and compliance to therapy. The dosage of the drug used for the methods according to the invention is preferably, in the case of human being, for example, about 0.1 mg to about 1, 000 mg per dose, in terms of siRNA molecules per kg of body weight for an adult. The concentration of the drug used for the treatment method according to the invention is preferably, in the case of siRNA, preferably 5 nM to 180 µM.

Regarding the route of administration, various routes including both oral and parenteral routes, for example, routes such as oral, intravenous, intramuscular, subcutaneous, topical, intratumoral, rectal, intraarterial, intraportal, intramedullary, intrapulpal, sublingual, intraoral, intraventricular, transmucosal, transdermal, intranasal, intraperitoneal, intrapulmonary, and intrauterine routes are included.

The frequency of administration varies depending on the properties of the agent or composition used, or the conditions of the subject including the above-described items; however, for example, the frequency of administration may be several times a day (that is, 2, 3, 4 times, or 5 or more times, a day), once a day, once in every several days (that is, every 2, 3, 4, 5, 6, or 7 days), once in every week, or once in several weeks (that is, once in 2, 3, or 4 weeks).

In the combination therapy according to the invention, an HSP47 inhibitor and a chemotherapeutic agent may be administered separately or may be administered simultaneously; however, in the case of administering the agents separately, it is preferable to administer the HSP47 inhibitor first. In a case where the chemotherapeutic agent is administered after the administration of the HSP47 inhibitor, the administration of the chemotherapeutic agent is preferably after 1 hour to after 70 hours, more preferably after 2 hours to after 60 hours, and particularly preferably after 4 hours to after 50 hours, from the administration of the HSP47 inhibitor. When the chemotherapeutic agent is administered after 4 hours to after 50 hours from the administration of the HSP47 inhibitor, cancer treatment by the chemotherapeutic agent can be achieved in a state in which decrease in the expression of HSP47 protein has been sufficiently exhibited, and therefore, the viability of cancer cells can be markedly decreased.

As used in the present specification, the term "patient" means any biological individual; however, the patient is, for example, an animal, a mammal, or a human individual. According to the invention, the term "patient" typically means a cancer patient who requires treatment with a chemotherapeutic agent.

Furthermore, the term "treatment" as used in the present specification is meant to include all kinds of medically acceptable prophylactic and/or therapeutic interventions intended for curing, transient remission or prevention of a disease, or the like. For example, the term "treatment" includes medically acceptable interventions for a variety of purposes, including delaying or stopping of the progression of a disease, involution or elimination of lesions, prevention of onset, prevention of recurrence, or the like.

This time, it has been disclosed by the inventors that as the medicine according to the invention suppresses the expression of transcript RNA of HSP47 (SEQ ID NO:1), the sensitivity to a chemotherapeutic agent can be enhanced in a cell line of breast cancer, colorectal cancer, colon cancer, or pancreatic cancer, and consequently, combination therapy for cancer is enabled.

Furthermore, as a result of the invention, the amount of administration of a chemotherapeutic agent can be reduced to a large extent in cancer treatment, and anticancer treatment can be carried out while reducing the risk of side effects caused by the chemotherapeutic agent.

Therefore, the invention also relates to a medicine for providing the above-described action, the medicine comprising an HSP47 inhibitor; use of an HSP inhibitor in the production of a medicine for providing the above-described action; and use of an HSP47 inhibitor for providing the above-described action.

### [Examples]

The invention will be described more specifically by way of the following Examples; however, the technical scope of the invention is not intended to be limited to these Examples.

### Example 1: Acquisition and culture of cell lines

All the human cancer cell lines (MDA-MB-231, HCT116, SW480, PANC-1, Suit2, and MIA-PaCa-2) used in the present specification were purchased from American Type Culture Collection. MDA-MB-231 cells, HCT116 cells, SW480 cells, Suit2 cells, and MIA-PaCa-2 cells were cultured in Dulbecco's Modified Eagle Medium (DMEM, Sigma-Aldrich, St. Louis, MO) supplemented with 10% fetal bovine serum (FBS, Invitrogen Life Technologies). PANC-1 cells were cultured in RPMI1640 medium supplemented with 10% FBS.

### Example 2: Transfection with siRNA

Cancer cells were transfected with control siRNA (siControl, Ambion, Foster City, CA), HSP47-A siRNA (siHSP47-A, sense: 5'-CUACGACGACGAGAAGGAAtt-3' (SEQ ID NO:2); antisense: 5'-UUCCUUCUCGUCGUCGUAGta-3' (SEQ ID NO:3)), HSP47-B siRNA (siHSP47-B, sense: 5'-AGCCCUCUUCUGACACUAAtt-3' (SEQ ID NO:4); antisense: 5'-UUAGUGUCAGAAGAGGGCUgg-3' (SEQ ID NO:5)), or HSP47-C siRNA (siHSP47-C, sense: 5'-GGACAGGCCUCUACAACUAtt-3' (SEQ ID NO:6) ; antisense: 5'-UAGUUGUAGAGGCCUGUCCtt-3 ' (SEQ ID NO:7)), using Lipofectamine RNAiMAX (Invitrogen Life Technologies), and the cancer cells were cultured for 5 hours at 37°C in the air. Five hours after the transfection with siRNA, the cells were cultured in DMEM supplemented with 10% FBS.

### Example 3: Establishment of HSP47 ^{-/-} cells

pCG SapI vector (acquired from Dr. Sakurai, Graduate School of Medicine, Shinshu University) includes cloning sites for inserting a Cas9 sequence (SEQ ID NO:9) and a target gRNA sequence (SEQ ID NO: 8) (Uemura et al., Sci Rep. 2016 Oct 26; 35861, Takei et al., Sci. Rep. 2017 Aug 24 7 (1) ; 9389). A gRNA sequence ( 5'-CCGACTGTACGGACCCAGCTCAG-3' (SEQ ID NO:8)) specifically designed for exon 2 of the human HSP47 genome was inserted into the pCG SapI vector. The targeting vector thus constructed and pcDNA3.1(+) were co-transfected into cancer cells (SW480, HCT116, PANC1, Suit2, MIA-PaCa-2, and MDA-MB-231) using Lipofectamine 2000 (Thermo Fisher Scientific). The cells were cultured in DMEM medium supplemented with 10% FBS and G418 (1000 (µg/mL), and cell lines that stably silenced HSP47 were selected. Single cell clones were evaluated by a DNA sequencing analysis, and indels (insertions and deletions) in target alleles were detected. Furthermore, the expression level of HSP47 protein in the selected clones was checked by Western blotting. Control cell clones (mock) were created by transfecting an empty vector.

### Example 4: Viability of cells treated with anticancer agent

The cancer cells transfected in Examples 2 and 3 were treated for 24 hours with cis-diaminedichloroplatinum(II) (CDDP, Sigma-Aldrich) at a concentration of 1, 5, 10, 20, or 50 µM, or with 5-fluorouracil (Wako Pure Chemical, Tokyo, Japan) at a concentration of 1, 5, 10, 20, or 50 µM. The viability of the treated cells was determined by a Dye-exclusion assay.

The results are shown in Figs. 1 to 3. All data are presented as mean ± standard deviation. Differences between groups were tested for statistical significance using Student' s t-test or analysis of variance (ANOVA) . Statistical significance was determined as P < 0.05.

It was found that the medicine according to the invention enhances the sensitivity of various cancer cells such as breast cancer, colorectal cancer, colon cancer, pancreatic adenocarcinoma, or pancreatic cancer to chemotherapeutic agents, by inhibiting the expression of HSP47.

In Fig. 1, when the concentration relative value of a chemotherapeutic agent required to induce a viability of cancer cells of 50% without using an HSP47 inhibitor (siControl group) was designated as 100, in a case where HSP47 inhibitors were used (siHSP47-A, siHSP47-B, and siHSP47-C), the relative values of chemotherapy were all less than 50, and sensitivity was markedly increased.

## Claims

1. A medicine for increasing sensitivity of a cancer patient to a chemotherapeutic agent, comprising an HSP47 inhibitor.

2. The medicine according to claim 1, wherein the HSP47 inhibitor is an interfering nucleic acid against HSP47, a ribozyme, an antisense nucleic acid, a microRNA, a short hairpin RNA, a vector expressing any of these, or a cell transformed with any of these.

3. The medicine according to claim 2, wherein the interfering nucleic acid against HSP47 is a siNA or a siRNA.

4. The medicine according to any one of claims 1 to 3, wherein the cancer patient is a patient with breast cancer, colorectal cancer, colon cancer, or pancreatic cancer.

5. The medicine according to any one of claims 1 to 4, wherein the chemotherapeutic agent is an alkylating agent, an antimetabolite, an antitumor antibiotic substance, an alkaloid, a hormonal therapeutic agent, a platinum complex, an angiogenesis inhibitory agent, a topoisomerase inhibitory agent, or a microtubule agonist.

6. The medicine according to any one of claims 1 to 5, wherein the chemotherapeutic agent is cisplatin (CDDP) or 5-fluorouracil (5-FU).

7. A medicine for treating cancer, comprising an HSP47 inhibitor and a chemotherapeutic agent.

8. Use of an HSP47 inhibitor in production of a medicine for the treatment of cancer, the treatment being a treatment of administering a chemotherapeutic agent and an HSP47 inhibitor in combination.

9. A composition comprising an HSP47 inhibitor for use in the treatment of cancer, the treatment being a treatment of administering a chemotherapeutic agent and an HSP47 inhibitor in combination.

10. A method for increasing sensitivity of a cancer patient to a chemotherapeutic agent, comprising administering an effective amount of an HSP47 inhibitor to a cancer patient.

11. The method according to claim 10, further comprising a step of identifying a patient in need of treatment using a medicine comprising an HSP47 inhibitor.

12. The method according to claim 11, wherein the step is a step of quantitatively determining the HSP47 expression in a tumor tissue isolated from a cancer patient.

13. A method for treating cancer, comprising administering effective amounts of an HSP47 inhibitor and a chemotherapeutic agent to a cancer patient.

14. A method for treating cancer cells in vitro, comprising treating cancer patient-derived cancer cells in vitro using effective amounts of an HSP47 inhibitor and a chemotherapeutic agent.
